(19) Europäisches Patentamt
European Patent Office
Office européen des brevets

(11) EP 1 165 091 B1

(12) # EUROPEAN PATENT SPECIFICATION

(45) Date of publication and mention of the grant of the patent:
**26.01.2005 Bulletin 2005/04**

(21) Application number: **00917940.9**

(22) Date of filing: **22.03.2000**

(51) Int Cl.[7]: **A61K 31/4433**, A61K 47/44

(86) International application number:
**PCT/US2000/006681**

(87) International publication number:
**WO 2000/057885 (05.10.2000 Gazette 2000/40)**

(54) **PHARMACEUTICAL EMULSIONS FOR RETROVIRAL PROTEASE INHIBITORS**

PHARMAZEUTISCHE EMULSIONEN FÜR RETROVIRAL-PROTEASE HEMMENDE VERBINDUNGEN

EMULSIONS PHARMACEUTIQUES POUR INHIBITEURS DE LA PROTEASE RETROVIRALE

(84) Designated Contracting States:
**AT BE CH CY DE DK ES FI FR GB GR IE IT LI LU MC NL PT SE**
Designated Extension States:
**AL LT LV MK RO SI**

(30) Priority: **31.03.1999 US 127026 P**

(43) Date of publication of application:
**02.01.2002 Bulletin 2002/01**

(73) Proprietor: **PHARMACIA & UPJOHN COMPANY
Kalamazoo, Michigan 49001 (US)**

(72) Inventors:
- **WANG, Youmin
Brookfield, WI 53045 (US)**
- **MOROZOWICH, Walter
Kalamazoo, MI 49002 (US)**

(74) Representative: **Perry, Robert Edward
GILL JENNINGS & EVERY
Broadgate House
7 Eldon Street
London EC2M 7LH (GB)**

(56) References cited:
**WO-A-99/06043** **WO-A-99/06044**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 165 091 B1

Printed by Jouve, 75001 PARIS (FR)

## Description

FIELD OF THE INVENTION

The present invention relates to novel pharmaceutical compositions in the form of emulsions with high oral bioavailability and prolonged blood levels for compounds which are inhibitors of retroviral protease. In particular, the composition is a safe emulsion formulation comprising a HIV protease inhibitor, an oil component, an emulsifying agent consisting of lecithin and solvents for pediatric patients.

BACKGROUND OF THE INVENTION

It has recently been discovered that certain pyranone compounds inhibit retroviral protease and thus they are useful for treating patients infected with human immunodeficiency virus (HIV) which results in acquired immunodeficiency syndrome (AIDS). In particular, the pyranone compound of formula I has been found to be especially effective as an inhibitor of retroviral protease.

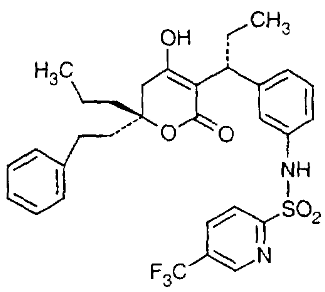

I

Several emulsion formulations of HIV drug are available in the prior art. However, these formulations require synthetic surfactants as emulsifiers to stabilize the compositions. For example, International publication WO 99/06043 discloses a self-emulsifying formulation which comprises the above pyranone compound of formula I, a mixture of diglyceride and monoglyceride, one or more solvents and one or more surfactants. Another example, WO 99/06044 discloses a discloses a self-emulsifying formulation which comprises the above pyranone compound of formula I, a basic amine, one or more solvents and one or more surfactants. Unfortunately, the catalog of suitable synthetic surfactants is limited. Although their potential scope is considerable, extensive toxicological studies are generally necessary to prove the harmlessness of the surfactants used in an emulsion formulation. Especially, the potentially adverse effect of surfactants on the newborn or small children is of major concern in today's society. Newborn and small children generally are more susceptible to chemical agents than adults.

Recognizing the potential problems, the present invention is directed toward pharmaceutical compositions in the form of emulsions which use natural or nontoxic lecithin as an emulsifier. The formulations of the present invention do not contain synthetic surfactants and, therefore, they are much safer for adult and pediatric use. It has also been discovered that lecithin dramatically increases the solubility of pyranone compound of formula I in oils. As a result, a high concentration of an emulsion formulation with high oral bioavailability can be prepared. Most surprisingly, the formulations of the present invention produce a prolonged blood level curve. Therefore, they have the potential for sustained release and high trough levels in the clinic.

INFORMATION DISCLOSURE

US Patent No. 5,852,195 discloses pyranone compounds of formula I useful to treat retroviral infections.

The International Publication No. WO 96/39142 discloses compositions which increase the bioavailability of protease inhibitors.

The International Publication No. WO 99/06043 discloses a self-emulsifying formulation which comprises the above pyranone compound of formula I, a mixture of diglyceride and monoglyceride, one or more solvents and one or more surfactants.

The International Publication No. WO 99/06044 discloses a discloses a self-emulsifying formulation which comprises the above pyranone compound of formula I, a basic amine, one or more solvents and one or more surfactants.

US patent No. 5,693,337 discloses a lipid emulsion which comprises a drug, an oil component, an emulsifying agent containing lecithin, water and citric acid.

US patent No. 5,665,700 discloses a pharmaceutical formulation comprising (a) a hydrophilic phase containing water, a biologically active material and lecithin; (b) a lipophilic phase containing one or more oils, a phospholipid and a lipophilic surfactant.

The International Publication No. WO 91/14429 discloses a composition for oral administration containing an active ingredient ipriflavone in combination with oily vehicles.

The International Publication No. WO 98/01118 discloses a pharmaceutical composition for oral delivery, including a liquid carrier oil, a homogenizing agent and a surfactant, an active agent and one or more excipients or carriers.

The International Publication No. WO 98/37869 discloses a O/W medical fat emulsion for oral administration which comprises an oil component, an emulsifier and a medicine as the indispensable constituents and dispersed in water.

The International Publication No. WO 98/22106 discloses a liquid pharmaceutical composition providing improved oral bioavailability for compounds which are inhibitors of HIV protease.

UK Patent application, GB2,257,359A discloses pharmaceutical compositions suitable for oral administration comprising a cyclosporin, 1,2-propylene glycol, a mixed mono-, di-, and tri-glyceride and a hydrophilic surfactant.

## SUMMARY OF THE INVENTION

One object of the present invention is to provide a safe pharmaceutical composition comprising the pyranone compound of formula I for HIV infected patients.

Another object of the present invention is to provide a safe pediatric formulation for HIV infected infants or small children who are especially vulnerable to the side effects of synthetic surfactants.

A further object of the present invention is to provide an emulsion formulation containing a high drug load of the pyranone compound of formula I for convenient administration which also has high oral bioavailability.

A still further object of the present invention is to provide an emulsion formulation which is capable of producing a prolonged blood level curve indicating sustained release.

The objects of the present invention have been accomplished in that the present invention provides an emulsion formulation containing natural, nontoxic lecithin as an emulsifier while at the same time allows a high loading of the pyranone compound of formula I. This novel formulation also achieves the desired high oral bioavailability and produces a prolonged blood level curve

The present invention specifically provides a pharmaceutical composition which comprises:

(a) a therapeutically effective amount of the compound of formula I,
(b) an oil component selected from the group consisting of mono-, di-, tri-glyceride or a mixture thereof wherein the monoglyceride and diglyceride are mono- and di-unsaturated fatty acid esters of glycerol having sixteen to twenty-two carbon atom chain length, wherein triglyceride is a saturated fatty acid ester of glycerol having six to twelve carbon atom chain length,
(c) an emulsifying agent consisting of lecithin, and
(d) a liquid phase comprising one or more pharmaceutically acceptable solvents.

## DETAILED DESCRIPTION OF THE INVENTION

In accordance with the present invention, there is a pharmaceutical composition comprising a pyranone compound as a pharmaceutically active agent in an emulsion vehicle.

For the purpose of the present invention, the term "pyranone compound" refers to compounds of formula I as defined above and its pharmaceutically acceptable salt thereof.

The compounds of formula I inhibit retroviral protease and thus inhibit the replication of the virus. They are useful for treating patients infected with human retrovirus, such as human immunodeficiency virus (strains of HIV-1 or HIV-2) or human T-cell leukemia viruses (HTLV-I or HTLV-II) which results in acquired immunodeficiency syndrome (AIDS) and/or related diseases. The compound of formula I is disclosed and claimed in U.S. Patent No. 5,852,195, and can be prepared according to the procedures described in said U.S. Patent.

The amount of active ingredient in the emulsion formulations of the present invention may vary or be adjusted widely depending on the intended route of administration, ages of patients being treated, the severity of the retroviral infection and the required concentration.

In preparing the emulsion formulations of the present invention, the amount of active ingredient is not particularly restricted up to 200 mg/ml (20% by weight) for maintaining high oral bioavailability, but preferably ranges from about 1% to about 20%, and more preferably from about 2% to about 15%, by weight of the total composition.

The emulsion formulations of the present invention may be administered orally, or parenterally at a dosage to obtain and maintain a concentration, that is, an amount, or blood-level of the active ingredient in the patients, which will be therapeutically effective. Generally, such therapeutically effective amount of active ingredient ranges from about 0.1 to about 300 mg/kg of body weight per day. Also, it is to be understood that the initial dosage administered may be increased beyond the above upper level in order to rapidly achieve the desired blood-level or the initial dosage may be smaller than the optimum and the daily dosage may be progressively increased during the course of treatment depending on the particular situation. If desired, the daily dose may also be divided into multiple doses for administration, e.g., two or more times per day.

The term "oil component" refers to monoglyceride, diglyceride, triglyceride, or a mixture comprising at lest two members of these three glycerides.

Preferably, the oil component is triglyceride, a mixture of diglyceride and monoglyceride in a ratio of from about 9:1 to about 1:9 by weight, a mixture of diglyceride and triglyceride in a ratio of from about 9:1 to about 1:9 by weight, or a mixture of monoglyceride, diglyceride and triglyceride in a ratio of from about 1 to about 8 parts of diglyceride per 10 parts of the mixture, about 1 to 5 parts of monoglyceride per 10 parts of the mixture, and about 1 to about 8 parts of triglyceride per 10 parts of the mixture.

More preferably, the oil component is triglyceride, a mixture of diglyceride and monoglyceride in a ratio of about 8:2 (diglyceride : monoglyceride) by weight, a mixture of diglyceride and triglyceride in a ratio of about 8:2 (triglyceride : diglyceride) by weight.

Diglyceride (hereinafter GDO of the present invention refers to a long chain fatty acid ester of glycerol having structural formula $HOCH_2$-$CH(O_2CR)$-$CH_2(O_2CR)$ or $(RCO_2)CH_2$-$CH(OH)$-$CH_2(O_2CR)$, wherein R is mono-unsaturated or di-unsaturated alkyl group having fifteen to twenty-one carbon atoms. The preferred diglyceride is diolein (R is mono-unsaturated alkyl group with seventeen carbon atoms), dilinoleate (R is di-unsaturated alkyl group with seventeen carbon atoms), or a mixture of diolein and dilinoleate. The most preferred diglyceride is diolein.

Monoglyceride (hereinafter GMO) of the present invention refers to a long chain fatty acid ester of glycerol having structural formula $HOCH_2$-$CH(OH)$-$CH_2(O_2CR)$ or $HOCH_2$-$CH(O_2CR)$-$CH_2OH$, wherein R is a mono-unsaturated or di-unsaturated alkyl group having fifteen to twenty-one carbon atoms. The preferred monoglyceride is monoolein (R is mono-unsaturated alkyl group with seventeen carbon atoms), monolinoleate (R is di-unsaturated alkyl group with seventeen carbon atoms), or a mixture of monoolein and monolinoleate. The most preferred monoglyceride is monoolein.

Triglyceride (hereinafter MCT) of the present invention refers to a medium chain fatty acid ester of glycerol having structural formula $R^1OCH_2$-$CH(O_2CR^1)$-$CH_2(O_2CR^1)$, wherein $R^1$ is a saturated alkyl group having five to eleven carbon atoms. The preferred triglyceride is under brand names Miglyol 810, Miglyol 812, Miglyol 829, etc. which refer to different grades of fractionated and purified coconut oil consisting mainly of medium chain triglycerides.

In preparing the emulsion of the present invention, the amount of the oil component is not particularly restricted, but preferably ranges from about 5% to about 40%, more preferably from about 10% to about 30%, and most preferably from about 10 % to about 20%, by weight of the total composition.

An oil component which comprises the mixture of diglyceride, monoglyceride or triglyceride may be prepared by mixing individual oil in appropriate proportion or by partial hydrolysis of triglyceride, or transesterification reaction of triglycerides or diglycerides with glycerol.

All of the glycerides of the present invention are known and can be obtained by conventional methods from a broad spectrum of water-immiscible material, such as soybean oil, avocado oil, squalene oil, sesame oil, olive oil, canola oil, corn oil, rapeseed oil, safflower oil, sunflower oil, coconut oil or mixtures thereof.

The term "lecithin" used herein refers monoaminomonophospholipid, a group of substances having structure formula FACOO-$CH_3$-CH(OOCFA)-$CH_2$-$OPO_4$-$NR''_3$, wherein FA is a fatty acid, R" is an alkyl radical. Lecithin also refers to esters of oleic, steric, palmitic, or other fatty acids with glycerolphosphoric acid and choline. Lecithin may be, for instance, yolk lecithin and soybean lecithin, or synthetic nontoxic didecanoyl phosphatidycholine, dilauroyl phosphatidycholine, dimyristoyl phosphatidycholine, dipalmitoyl phosphatidycholine or a mixture thereof. Therefore, lecithin is not restricted to specific ones so far as they have compositions comprising the foregoing element. Lecithin is used as a emulsifying agent in the present invention. It also operates as a solubilizing agent.

In preparing the lipid emulsion of the present invention, the amount of lecithin to be used is not particularly restricted, but preferably ranges from about 0.5% to about 20%, more preferably from about 1% to about 10% and most preferably from about 2% to about 5%, by weight of the total composition.

Solvents of the present invention refer to propylene glycol, polyethylene glycol, glycerol, triacetin, dimethyl isosorbide, glycofurol, propylene carbonate, ethanol, water, dimethyl acetamide or a mixture thereof. The preferred solvent is propylene glycol, ethanol, water or a mixture thereof. The most preferred solvent is water.

The term "q.s." used herein refers to quantum sufficit or as much as being sufficient.

In the preparation of the lipid emulsion of the present invention, the amount of solvent(s) is not particularly restricted, but preferably ranges from about 20% to 95% by weight of the total composition.

If desired, the compositions of the present invention may further comprise conventional pharmaceutical additives such as coloring agents, flavoring agents, thickening agents, stabilizers such as sodium deoxycholate, antioxidants such as BHT or vitamin E, and preserving agents such as methyl paraben, or propyl paraben.

The compositions of the present invention may be prepared in a conventional manner, for example, (1) preparing an oil phase by dissolving an active ingredient in a mixture of an oil component, solvent(s), lecithin and optional water-immiscible excipients by heat, (2) preparing an aqueous phase by dissolving optional excipients in water, (3) combining the oil phase and the aqueous phase using a high energy homogenizer to achieve a submicron lipid emulsion.

The pharmaceutical compositions of the present invention will be better understood in connection with the following examples, which are intended as an illustration of and not a limitation upon the scope of the invention. Without further elaboration, it is believed that one skilled in the art can, using the preceding description and the information provided in the examples below, practice the present invention to its fullest extent.

EXAMPLE 1

| Component | Weight (mg) |
|---|---|
| Compound of Formula I | 60 |
| Miglyol 812 | 200 |
| Propylene Glycol | 100 |
| Lecithin | 20 |
| Na Deoxycholate | 0.5 |
| glycerine | 24 |
| Methyl paraben | 1.8 |
| Propyl paraben | 0.2 |
| Water | q.s. |

EXAMPLE 2

| Component | Weight (mg) |
|---|---|
| Compound of Formula I | 60 |
| GDO/GMO (8:2) | 200 |
| Propylene Glycol | 100 |
| Lecithin | 20 |
| Na Deoxycholate | 0.5 |
| glycerine | 2.4 |
| Methyl paraben | 1.8 |
| Propyl paraben | 0.2 |
| Water | q.s. |

EXAMPLE 3

| Component | Weight (mg) |
|---|---|
| Compound of Formula I | 60 |
| Miglyol 812 | 200 |
| Propylene Glycol | 100 |
| Lecithin | 20 |

(continued)

| Component | Weight (mg) |
|---|---|
| Na Deoxycholate | 0.5 |
| glycerine | 24 |
| Methyl paraben | 1.8 |
| Propyl paraben | 0.2 |
| Water | q.s. |

The examples 1 and 3 are the same formulation but different particle sizes

EXAMPLE 4

| Component | Weight (mg) |
|---|---|
| Compound of Formula I | 60 |
| GDO | 200 |
| Propylene Glycol | 100 |
| Lecithin | 20 |
| Na Deoxycholate | 0.5 |
| glycerine | 2.4 |
| Methyl paraben | 1.8 |
| Propyl paraben | 0.2 |
| Water | q.s. |

EXAMPLE 5

| Component | Weight (mg) |
|---|---|
| Compound of Formula I | 60 |
| MCT/GDO (8:2) | 200 |
| Lecithin | 20 |
| BHT | 0.1 |
| glycerine | 2.4 |
| Water | q.s. |

Oral Bioavailability Test.

(i) Sprague-Dawley male rats were selected for the *in vivo* oral bioavailability study. Each rat was prepared by the surgical implantation of an indwelling cannula in the superior vena cava. Each rat, in the weight range of 300 - 400 g, was fasted overnight prior to dosing. Each formulation was orally administered to a group of rats (n=6) at a 20 mg/kg dose. The formulations with high concentration of the compound of formula I (typically 50 mg/g) was diluted by 25-fold with water and injected directly into the rat's stomach using oral gavage. Serial blood samples of 0.25 ml were obtained from the indwelling cannula at 0.25, 0.5, 1, 2, 4, 6, 8, 12, and 24 hours after dosing. These blood samples were analyzed using a HPLC assay specific for the compound of formula I. Drug concentrations in the blood of the test rats are plotted against the time after the drug is administered through an intravenous (i.v.) or oral route and the AUCs (the Area Under the Pasma Concentration-Time Curve) are integrated using the trapezoidal rule to calculate the absolute bioavailability as shown in Table 1.

$$\textit{Absolute bioavailability (F)} = \frac{(AUC)_{oral} / Dose_{oral}}{(AUC)_{iv} / Dose_{iv}}$$

(ii) Male Beagle dogs were also selected for the *in vivo* oral bioavailability study. Each dog, in the weight range of 13.5 - 17.5 kg, was fasted overnight and a light snack was given 30 minutes prior to dosing. Dogs were fed with regular dog food 4 hours after dosing. Each formulation was orally administered to a group of dogs (n=5) at a 20 mg/kg dose. The formulations of the compound of formula I (50 mg/g) were encapsulated in gelatin capsules and administered. Serial blood samples of 2 ml were obtained from the jugular vein at 20, 40 minutes and 1, 2, 4, 6, 8, 12, and 24 hours after dosing. These blood samples were analyzed using a HPLC assay specific for the compound of formula I. The blood concentrations of the compound of formula I are plotted against the time and the AUCs are obtained to calculate the absolute bioavailability. The results are shown in Table 2.

TABLE 1

| Absolute Mean Oral Bioavailability in Rats | |
|---|---|
| Example No. | Absolute Mean Oral Bioavailability (%) |
| 1 | 36 |
| 2 | 24 |
| 3 | 40 |
| 4 | 34 |
| Aqueous suspension of free acid of the compound of formula I | < 20 |

TABLE 2

| Absolute Mean Oral Bioavailability in Dogs | |
|---|---|
| Example No. | Absolute Mean Oral Bioavailability (%) |
| 1 | 31.4 |
| 2 | 33.0 |
| Free Acid of the compound formula I powder in Hard Gelatin Capsules | 1.5 |

**Claims**

**1.** A pharmaceutical composition comprising:

(a) a therapeutically effective amount of the pyranone compound of formula I

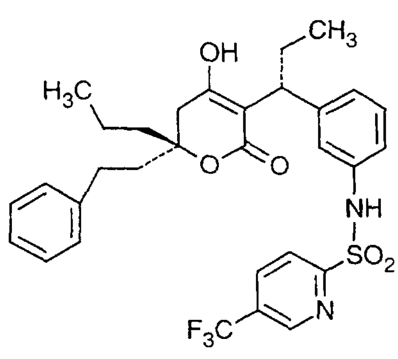

I

(b) an oil component selected from the group consisting of mono-, di-, tri-glyceride or a mixture thereof wherein the monoglyceride and diglyceride are mono- and di-unsaturated fatty acid esters of glycerol having sixteen to twenty-two carbon atom chain length, wherein triglyceride is a saturated fatty acid ester of glycerol having six to twelve carbon atom chain length,
(c) an emulsifying agent consisting of lecithin, and
(d) a liquid phase comprising one or more pharmaceutically acceptable solvents.

**2.** A pharmaceutical composition of claim 1 wherein the pyranone compound of formula I is in an amount of from about 1% to about 20% by weight of the total composition.

**3.** A pharmaceutical composition of claim 1 wherein the pyranone compound of formula I is in an amount of from about 2% to about 15% by weight of the total composition.

**4.** A pharmaceutical composition of claim 1 wherein the lecithin is yolk lecithin or soybean lecithin.

**5.** A pharmaceutical composition of claim 1 wherein the lecithin is a synthetic nontoxic didecanoyl phosphatidycholine, dilauroyl phosphatidycholine, dimyristoyl phosphatidycholine, dipalmitoyl phosphatidycholine or a mixture thereof.

**6.** A pharmaceutical composition of claim 1 wherein the lecithin is in an amount of from about 0.5% to about 20% by weight of the total composition.

**7.** A pharmaceutical composition of claim 1 wherein the lecithin is in an amount of from about 1% to about 10% by weight of the total composition.

**8.** A pharmaceutical composition of claim 1 wherein the lecithin is in an amount of from about 2% to about 5% by weight of the total composition.

**9.** A pharmaceutical composition of claim 1 wherein the oil component is in an amount of from about 5% to about 40% by weight of the total composition.

**10.** A pharmaceutical composition of claim 1 wherein the oil component is in an amount of from about 10% to about 30% by weight of the total composition.

**11.** A pharmaceutical composition of claim 1 wherein the oil component is in an amount of from about 10% to about 20% by weight of total composition.

**12.** A pharmaceutical composition of claim 1 wherein the oil component is triglyceride.

**13.** A pharmaceutical composition of claim 1 wherein the oil component is a mixture of diglyceride and monoglyceride in a ratio of from about 9:1 to about 1:9 by weight.

**14.** A pharmaceutical composition of claim 1 wherein the oil component is a mixture of diglyceride and monoglyceride in a ratio of about 8:2 (diglyceride : monoglyceride) by weight.

**15.** A pharmaceutical composition of claim 1 wherein the oil component is a mixture of diglyceride and triglyceride in a ratio of from about 9:1 to about 1:9 by weight.

**16.** A pharmaceutical composition of claim 1 wherein the oil component is a mixture of diglyceride and triglyceride in a ratio of about 2:8 (diglyceride : triglyceride) by weight.

**17.** A pharmaceutical composition of claim 1 wherein the oil component is a mixture of monoglyceride, diglyceride and triglyceride in a ratio of from about 1 to about 8 parts of diglyceride per 10 parts of the mixture, about 1 to 5 parts of monoglyceride per 10 parts of the mixture, and about 1 to about 8 parts of triglyceride per 10 parts of the mixture.

**18.** A pharmaceutical composition of claim 1 wherein the pharmaceutically acceptable solvent is propylene glycol, polyethylene glycol, glycerol, triacetin, dimethyl isosorbide, glycofurol, propylene carbonate, ethanol, water, dimethyl acetamide or a mixture thereof.

**19.** A pharmaceutical composition of claim 1 wherein the pharmaceutically acceptable solvent is propylene glycol, water or a mixture thereof.

**20.** A pharmaceutical composition of claim 1 wherein the pharmaceutically acceptable solvent is water.

**21.** A pharmaceutical composition of claim 1 which is administered orally or parenterally.

**Patentansprüche**

**1.** Pharmazeutische Zusammensetzung, die umfasst:

(a) eine therapeutisch wirksame Menge der Pyranonverbindung der Formel (I)

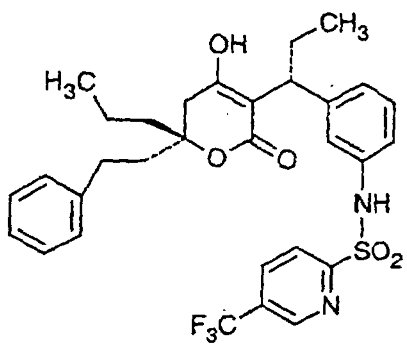

I

(b) eine Ölkomponente, die aus der aus Mono-, Di-, Triglycerid oder einem Gemisch derselben bestehenden Gruppe ausgewählt ist, wobei das Monoglycerid und Diglycerid Ester von einfach und zweifach ungesättigten Fettsäuren mit Glycerin mit einer Kettenlänge von 16 bis 22 Kohlenstoffatomen sind, wobei das Triglycerid ein Ester einer gesättigten Fettsäure mit Glycerin mit einer Kettenlänge von 6 bis 12 Kohlenstoffatomen ist,
(c) einen aus einem Lecithin bestehenden Emulgator und
(d) eine flüssige Phase, die ein oder mehrere pharmazeutisch akzeptable Lösemittel umfasst.

**2.** Pharmazeutische Zusammensetzung nach Anspruch 1, wobei die Pyranonverbindung der Formel I in einer Menge von etwa 1 bis etwa 20 Gew.-% der Gesamtzusammensetzung vorhanden ist.

**3.** Pharmazeutische Zusammensetzung nach Anspruch 1, wobei die Pyranonverbindung der Formel I in einer Menge von etwa 2 bis etwa 15 Gew.-% der Gesamtzusammensetzung vorhanden ist.

**4.** Pharmazeutische Zusammensetzung nach Anspruch 1, wobei das Lecithin Dotterlecithin oder Sojalecithin ist.

**5.** Pharmazeutische Zusammensetzung nach Anspruch 1, wobei das Lecithin ein synthetisches nicht-toxisches Didecanoylphosphatidylcholin, Dilauroylphosphatidylcholin, Dimyristoylphosphatidylcholin, Dipalmitoylphosphatidylcholin oder ein Gemisch derselben ist.

**6.** Pharmazeutische Zusammensetzung nach Anspruch 1, wobei das Lecithin in einer Menge von etwa 0,5 bis etwa 20 Gew.-% der Gesamtzusammensetzung vorhanden ist.

**7.** Pharmazeutische Zusammensetzung nach Anspruch 1, wobei das Lecithin in einer Menge von etwa 1 bis etwa 10 Gew.-% der Gesamtzusammensetzung vorhanden ist.

**8.** Pharmazeutische Zusammensetzung nach Anspruch 1, wobei das Lecithin in einer Menge von etwa 2 bis etwa 5 Gew.-% der Gesamtzusammensetzung vorhanden ist.

**9.** Pharmazeutische Zusammensetzung nach Anspruch 1, wobei die Ölkomponente in einer Menge von etwa 5 bis

etwa 40 Gew.-% der Gesamtzusammensetzung vorhanden ist.

**10.** Pharmazeutische Zusammensetzung nach Anspruch 1, wobei die Ölkomponente in einer Menge von etwa 10 bis etwa 30 Gew.-% der Gesamtzusammensetzung vorhanden ist.

**11.** Pharmazeutische Zusammensetzung nach Anspruch 1, wobei die Ölkomponente in einer Menge von etwa 10 bis etwa 20 Gew.-% der Gesamtzusammensetzung vorhanden ist.

**12.** Pharmazeutische Zusammensetzung nach Anspruch 1, wobei die Ölkomponente ein Triglycerid ist.

**13.** Pharmazeutische Zusammensetzung nach Anspruch 1, wobei die Ölkomponente ein Gemisch aus Diglycerid und Monoglycerid in einem Gewichtsverhältnis von etwa 9:1 bis etwa 1:9 ist.

**14.** Pharmazeutische Zusammensetzung nach Anspruch 1, wobei die Ölkomponente ein Gemisch aus Diglycerid und Monoglycerid in einem Gewichtsverhältnis von etwa 8:2 (Diglycerid:Monoglycerid) ist.

**15.** Pharmazeutische Zusammensetzung nach Anspruch 1, wobei die Ölkomponente ein Gemisch aus Diglycerid und Triglycerid in einem Gewichtsverhältnis von etwa 9:1 bis etwa 1:9 ist.

**16.** Pharmazeutische Zusammensetzung nach Anspruch 1, wobei die Ölkomponente ein Gemisch aus Diglycerid und Triglycerid in einem Gewichtsverhältnis von etwa 2:8 (Diglycerid:Triglycerid) ist.

**17.** Pharmazeutische Zusammensetzung nach Anspruch 1, wobei die Ölkomponente ein Gemisch aus Monoglycerid, Diglycerid und Triglycerid in einem Verhältnis von etwa 1 bis etwa 8 Teilen Diglycerid pro 10 Teile des Gemischs, etwa 1 bis 5 Teilen Monoglycerid pro 10 Teile des Gemischs und etwa 1 bis 8 Teilen Triglycerid pro 10 Teile des Gemischs ist.

**18.** Pharmazeutische Zusammensetzung nach Anspruch 1, wobei das pharmazeutisch akzeptable Lösemittel Propylenglykol, Polyethylenglykol, Glycerin, Triacetin, Dimethylisosorbid, Glykofurol, Propylencarbonat, Ethanol, Wasser, Dimethylacetamid oder ein Gemisch derselben ist.

**19.** Pharmazeutische Zusammensetzung nach Anspruch 1, wobei das pharmazeutisch akzeptable Lösemittel Propylenglykol, Wasser oder ein Gemisch derselben ist.

**20.** Pharmazeutische Zusammensetzung nach Anspruch 1, wobei das pharmazeutisch akzeptable Lösemittel Wasser ist.

**21.** Pharmazeutische Zusammensetzung nach Anspruch 1, die oral oder parenteral verabreicht wird.

## Revendications

**1.** Composition pharmaceutique comprenant :

(a) une quantité thérapeutiquement efficace du dérivé du pyranone de formule I

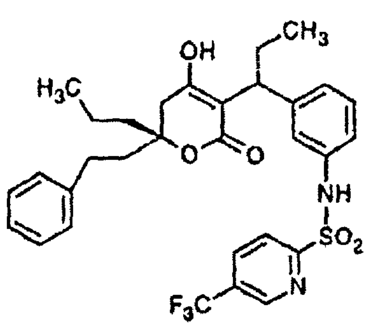

(I)

(b) un constituant huileux choisi dans le groupe consistant en des mono-, di- et triglycérides ou un de leurs mélanges, dans lequel le monoglycéride et le diglycéride sont des mono- et diesters d'acides gras insaturés du glycérol ayant une longueur de chaîne de seize à vingt-deux atomes de carbone, le triglycéride est un ester d'acide gras saturé du glycérol ayant une longueur de chaîne de six à douze atomes de carbone,
(c) un agent émulsionnant consistant en lécithine, et
(d) une phase liquide comprenant un ou plusieurs solvants pharmaceutiquement acceptables.

**2.** Composition pharmaceutique suivant la revendication 1, dans laquelle le dérivé de pyranone de formule I est présent en une quantité d'environ 1 % à environ 20 % en poids de la composition totale.

**3.** Composition pharmaceutique suivant la revendication 1, dans laquelle le dérivé de pyranone de formule I est présent en une quantité d'environ 2 % à environ 15 % en poids de la composition totale.

**4.** Composition pharmaceutique suivant la revendication 1, dans laquelle la lécithine est la lécithine de jaune d'oeuf ou la lécithine de soja.

**5.** Composition pharmaceutique suivant la revendication 1, dans laquelle la lécithine est une didécanoylphosphatidycholine dilauroylphosphatidycholine, dimyristoylphosphatidycholine, dipalmitoylphosphatidycholine non toxique synthétique ou un de leurs mélanges.

**6.** Composition pharmaceutique suivant la revendication 1, dans laquelle la lécithine est présente en une quantité d'environ 0,5 % à environ 20 % en poids de la composition totale.

**7.** Composition pharmaceutique suivant la revendication 1, dans laquelle la lécithine est présente en une quantité d'environ 1 % à environ 10 % en poids de la composition totale.

**8.** Composition pharmaceutique suivant la revendication 1, dans laquelle la lécithine est présente en une quantité d'environ 2 % à environ 5 % en poids de la composition totale.

**9.** Composition pharmaceutique suivant la revendication 1, dans laquelle le constituant huileux est présent en une quantité d'environ 5 % à environ 40 % en poids de la composition totale.

**10.** Composition pharmaceutique suivant la revendication 1, dans laquelle le constituant huileux est présent en une quantité d'environ 10 % à environ 30 % en poids de la composition totale.

**11.** Composition pharmaceutique suivant la revendication 1, dans laquelle le constituant huileux est présent en une quantité d'environ 10 % à environ 20 % en poids de la composition totale.

**12.** Composition pharmaceutique suivant la revendication 1, dans laquelle le constituant huileux est un triglycéride.

**13.** Composition pharmaceutique suivant la revendication 1, dans laquelle le constituant huileux est un mélange d'un diglycéride et d'un monoglycéride dans un rapport compris dans l'intervalle d'environ 9:1 à environ 1:9 en poids.

**14.** Composition pharmaceutique suivant la revendication 1, dans laquelle le constituant huileux est un mélange d'un diglycéride et d'un monoglycéride en un rapport d'environ 8:2 (diglycéride:monoglycéride) en poids.

**15.** Composition pharmaceutique suivant la revendication 1, dans laquelle le constituant huileux est un mélange d'un diglycéride et d'un triglycéride en un rapport compris dans l'intervalle d'environ 9:1 à environ 1:9 en poids.

**16.** Composition pharmaceutique suivant la revendication 1, dans laquelle le constituant huileux est un mélange d'un diglycéride et d'un triglycéride en un rapport d'environ 2:8 (diglycéride:triglycéride) en poids.

**17.** Composition pharmaceutique suivant la revendication 1, dans laquelle le constituant huileux est un mélange d'un monoglycéride, d'un diglycéride et d'un triglycéride en un rapport d'environ 1 à environ 8 parties de diglycéride pour 10 parties du mélange, d'environ 1 à 5 parties de monoglycéride pour 10 parties du mélange et d'environ 1 à environ 8 parties de triglycéride pour 10 parties du mélange.

**18.** Composition pharmaceutique suivant la revendication 1, dans laquelle le solvant pharmaceutiquement acceptable

est le propylèneglycol, le polyéthylèneglycol, le glycérol, la triacétine, le diméthylisosorbide, le glycofurol, le carbonate de propylène, l'éthanol, l'eau, le diméthylacétamide ou un de leurs mélanges.

**19.** Composition pharmaceutique suivant la revendication 1, dans laquelle le solvant pharmaceutiquement acceptable est le propylèneglycol, l'eau ou un de leurs mélanges.

**20.** Composition pharmaceutique suivant la revendication 1, dans laquelle le solvant pharmaceutiquement acceptable est l'eau.

**21.** Composition pharmaceutique suivant la revendication 1, qui est administrée par voie orale ou parentérale.